# EUROPEAN PATENT APPLICATION

(11) **EP 1 416 050 A1**
(43) Date of publication of application: **06.05.2004**
(21) Application number: 02738904.8
(22) Date of filing: 02.07.2002
(51) Int. Cl.: C12N 15/53, C12N 9/06, C12N 1/21, C12P 7/62

(54) **METHOD OF MODIFYING ENZYME AND OXIDOREDUCTASE VARIANT**

(30) Priority: 02.07.2001 JP 2001200417; 15.01.2002 JP 2002006303
(71) Applicant: KANEKA CORPORATION, Osaka-shi, Osaka 530-8288 (JP)
(72) Inventor: NAKAI, Takahisa, Kobe-shi, Hyogo 655-0853 (JP); MORIKAWA, Souichi, Himeji-shi, Hyogo 670-0001 (JP); KIZAKI, Noriyuki, Moriguchi-shi, Osaka 570-0016 (JP); YASOHARA, Yoshihiko, Himeji-shi, Hyogo 670-0942 (JP)
(74) Representative: HOFFMANN - EITLE
(86) International application number: PCT/JP2002/006688
(87) International publication number: WO 2003/004653

(57) **Abstract**

An enzyme modifying method for converting the coenzyme-dependency of an oxidoreductase is developed. Using this method, a novel carbonyl reductase mutant capable of utilizing NADH as a coenzyme is provided. It is also intended to provide a process for enzymatically producing an optically active (S)-4-halo-3-hydroxybutyric ester by utilizing the carbonyl reductase mutant.

A method for modifying an enzyme itself so as to convert the coenzyme-dependency of a carbonyl reductase which asymmetrically reduces a carbonyl compound to produce an optically active alcohol, a carbonyl reductase having such coenzyme dependency as has been converted from NADPH to NADH which is obtained by the above method, a DNA encoding this enzyme mutant, a plasmid carrying this DNA, a transformant obtained by the transformation with this plasmid, and a process for producing an optically active alcohol by using this enzyme mutant and/or this transformant.

## Description

### Field of Technology

This invention relates to a method for modifying the coenzyme dependency of an oxidoreductase, particularly a method for modifying the coenzyme dependency of an enzyme having an activity of asymmetrically reducing a carbonyl compound to produce an optically active alcohol (hereinafter referred to as CRD enzyme), wherein the enzyme dependency involves the conversion of reduced type β-nicotinamide adenine dinucleotide phosphate (hereinafter abbreviated to NADPH) to reduced type β-nicotinamide adenine dinucleotide (hereinafter abbreviated to NADH). This invention further relates to a process for producing a CRD enzyme mutant having NADH dependency obtained by said modification method, a DNA encoding said enzyme mutant, a plasmid carrying this DNA, a transformed cell obtained by transformation with this plasmid, and said enzyme. This invention also relates to a process for producing an optically active alcohol using the aforementioned enzyme and this transformed cell.

### Background Technology

An optically active (S)-4-halo-3-hydroxybutyric ester is a compound to be utilized for an intermediate for production of a medicine or the like. The method of production of a symmetric, optically pure version thereof is an important subject with industrial applications. Therefore, process development is enthusiastically explored in order to produce an optically active alcohol such as (S)-4-halo-3-hydroxybutyric ester or the like using a CRD enzyme, which asymmetrically reduces a carbonyl compound such as 4-haloacetoacetic ester or the like.

As a CRD enzyme producing (S)-4-halo-3-hydroxybutyric ester from 4-haloacetoacetic ester, 3a-hydroxysteroid dehydrogenase (JP Patent Publication (Kokai) No.1-277494 A (1989)), glycerol dehydrogenase [*Tetrahedron Lett.* 29, 2453 - 2454 (1988)], an alcohol dehydrogenase originated from *Pseudomonas sp.* PED [*J. Org. Chem.* 57, 1526 - 1532 (1992)], a ketopantotenic ester reductase originated from *Candida* *macedoniensis* [*Arch. Biochem. Biophys.* 294, 469 - 474 (1992)], a 4-chloroacetoacetic ethyl ester reductase derived from *Geotrichum candidum* [*Enzyme Microb. Technol*. 14, 731 - 738 (1992)], a carbonyl reductase originated from *Kluyveromyces lactis* (JP Patent Application No. 9-357969 (1997)), a carbonyl reductase originated from *Kluyveromyces aestuarii* (JP Patent Publication (Kokai) No. 2000-236883 A), etc. have been reported.

Among these enzymes, there are cases where not only reduction but also oxidation (dehydrogenation) occurs that causes problems, such as lowering optical purity and accumulation concentration of (S)-4-halo-3-hydroxybutyric ester as a product. By the use of a CRD enzyme derived from *Candida magnoliae* (hereinafter referred to as CMCRD enzyme) reported by a part of the present inventors, a process for producing a practical (S)-4-halo-3-hydroxybutyric ester was first provided (WO 98/35025). This CMCRD enzyme has excellent thermostability and organic solvent resistance and has satisfactorily high enzyme activity. However, because it is a reductase using NADPH as a coenzyme, it is considered to be desirable that another inexpensive, chemically stable coenzyme, e.g., NADH, be utilized instead of expensive, chemically unstable NADPH in order to establish a economical process for producing (S)-4-halo-3-hydroxybutyric ester.

Generally, in the production of optically active alcohols using an oxidoreductase, efficient regeneration of a coenzyme which the oxidoreductaseutilizes, is also an important matter. As a method for regenerating pyridine dinucleotide coenzyme such as β-nitotinamide adenine dinucleotide phosphate (hereinafter referred to as NADP), β-nitotinamide adenine dinucleotide (hereinafter referred to as NAD) and the like, for example, a method using a microorganism or a treated microorganism containing, for example, glucose dehydrogenase (hereinafter referred to as GDH), formic acid dehydrogenase (hereinafter referred to as FDH), alcohol dehydrogenase, amino acid dehydrogenase, organic acid dehydrogenase (such as malate dehydrogenase), or the like and a partially or fully purified enzyme is known. Here, there is a certain limitation in that the combination of enzymes which regenerates a coenzyme depends upon the type of the coenzyme. For example, GDH includes types which can regenerate NADP and types which can regenerate NAD, but FDH can regenerate NAD alone.

That is, it is considered that, if the coenzyme dependency of an enzyme having excellent properties (stability, solvent tolerance, oxidation tolerance) and a specific activity such as CMCRD enzyme can be optimized to have characteristics of coenzyme dependency of an enzyme which regenerates said coenzyme, the optimization of the process in the production of an optically active alcohol or the like becomes easy and a more efficient production process can be provided. Although it takes an enormous amount of labor to screen for an enzyme having excellent properties, a specific activity and desirable substrate specificity and coenzyme dependency, it becomes possible to promptly and efficiently improve and establish a production process if the functions such as coenzyme dependency, substrate specificity, etc. of an already known excellent enzyme can be freely controlled.

Until now, attempts to modify the substrate-specificity and the coenzyme-dependency of an enzyme have been made, and, as examples of the conversion of the coenzyme-dependency of oxidoreductase, reports relating to enzymes such as lactate dehydrogenase originating from *Bacillus stearothermophilus* [*Biochem. Biophys. Res. Commun*. 166, 667-672 (1990)], glutathione reductase originating from *Escherichia coli* [*Nature*, 343, 38-43 (1990)], isocitrate dehydrogenase originating from *Escherichia coli* [*Proc. Natl. Acad. Sci. USA,* 92, 11666-11670 (1995)], isopropyl malate dehydrogenase originating from *Thermus thermophilus* [*Proc. Natl. Acad. Sci. USA,* 93, 12171-12176 (1996)], HMG-CoA reductase originated from *Pseudomonas mevalonii* [*Biochemistry*, 55, 11945-11950 (1996)], leucine dehydrogenase originating from *Thermoactinomyces intermedius* [*Protein. Eng*. 10,687-690 (1997)], p-hydroxybenzoic acid hydroxylase originated from *Pseudomonas fluorescence* [*J. Mol. Biol*. 292, 87-96 (1999)], etc. have been made.

However, the conversion in many of these reports was carried out by trial-and-error amino acid mutation, centering on information about the similarity of sequences obtained from amino acid sequences. Generally, the functions and physicochemical properties of an enzyme are closely related to three dimensional structure, therefore, it must be said that a trial-and-error amino acid mutation method centering on the information about the similarity of a sequence obtained from amino acid sequences is an extremely inefficient method when introducing into an enzyme modification that strictly controls the reactivity.

Although there are enzyme modification reports concerning the design of rational amino acid mutation based on the three dimensional structure of an enzyme, many of them adopt a method in which modification is carried out by substituting the coenzyme binding site of an analogous enzyme having desired coenzyme dependency (a technique involving the preparation of a chimera enzyme), presuming a residue participating in binding based on the three dimensional structure of said analogous enzyme and selecting an amino acid residue to be substituted based on the similarity to the amino acid sequence of the analogous enzyme. In such cases, a criterion for selecting an amino acid residue to be substituted depends upon the designers' experiences, proclivities, and opinions. In addition, estimation of the contribution of each specified amino acid residue based on objective indications was not carried out, thus, these are hardly truly rational enzyme modification design. In case that the obtained mutant does not have any desired functions or has insufficient functions, there is a necessity of repeating trial-and-error experiments again. Therefore, it is considered to be extremely inefficient method.

Because of such technical problems, although some changes can be found in coenzyme-dependency via the conventional modification methods, there still remain problems such as the obtained modified enzyme does not have any practically sufficient enzyme activities, or, for example, the coenzyme-dependency cannot be controlled strictly because obtained mutant can utilize both NADP and NAD as coenzymes. Therefore, the acquisition of a practical modified enzyme that has industrial applications has not been achieved. It is desired to develop a rational designing method for enzyme modification effective for the solution of these problems, preferably an objective automated design technique.

### Disclosure of Invention

This invention purposes to provide a rational enzyme modification method for converting the coenzyme dependency of an oxidoreductase in order to solve the problems described above. This invention further purposes to provide a CRD enzyme mutant which can utilize NADH as a coenzyme according to said modification method, a DNA encoding said CRD enzyme mutant and a recombinant into which the DNA is introduced, and a process for producing an optically active (S)-4-halo-3-hydroxybutyric ester using the same.

As a result of intensive studies for solving the above problems, the present inventors developed an enzyme modification method converting the coenzyme-dependency of an oxidoreductase, succeeded in preparation of a CRD enzyme mutant which can utilize NADH as a coenzyme according to said enzyme modification method, and completed the present invention.

That is, the present invention relates to a method for modifying an enzyme in order to convert the coenzyme-dependency of an oxidoreductase, characterized in that the size of binding energy of a coenzyme molecule is controlled by the substitution, insertion, deletion or the combination thereof of a single or plural arbitrary amino acid residue at the previously selected site of said oxidoreductase.

As a preferable embodiment of the present invention, the above enzyme modification method includes a step of identifying an active site of an oxidoreductase, a step of determining an amino acid residue interacting with a coenzyme molecule in the neighborhood of said active site, and a step of carrying out mutation treatment in order to control the size of the binding energy of the coenzyme molecule over said determined residue.

As a further preferable embodiment of the present invention, the aforementioned step of specifying the active site in the above enzyme modification method further includes treatments of predicting the three dimensional structure by molecule modeling method, searching for the cleft part having a volume capable of accommodating a coenzyme molecule, comparing amino acid sequences of the enzyme with an analogous enzyme protein, and extracting residues presumed to be important for the binding of enzyme and coenzyme molecules from the amino acid residues constituting said cleft part.

As another preferable embodiment of the present invention, the oxidoreductase in the above enzyme modification method is the one utilizing pyridine nucleotide coenzyme as a coenzyme molecule.

As another preferable embodiment of the present invention, the oxidoreductase in the above enzyme modification method is a carbonyl oxidoreductase, particularly preferably the one derived from *Candida magnoliae* IFO 0705.

In addition, the present invention relates to a carbonyl oxidoreductase mutant, which is obtained from a wild-type carbonyl oxidoreductase by substitution, insertion, deletion or the combination thereof of an amino acid residue and which has the following physicochemical properties:
(1) Action:
   Acting on 4-chloroacetoacetic acid ethyl to produce ethyl (S)-4-chloro-3-hydoroxybutyrate by using NADH as a coenzyme;
(2) Substrate Specificity:
   Showing a strong activity to ethyl 4-chloroacetoacetate but substantially no activity to ethyl acetoacetate,
   showing a strong activity to 4-chloroacetoacetic ester but substantially no dehydrogenase activity to 4-halo-3-hydroxybutyric ester; and
(3) Coenzyme dependency:
   Showing a strong activity in case of serving NADH as a coenzyme but substantially no activity in case of serving NADPH as a coenzyme;

   Further preferably to a carbonyl oxidoreductase mutant having the following physicochemical properties of (4) - (7):
(4) Optimal pH: 4.0 - 7.0;
(5) Thermostability: Stable until 45°C in case of the treatment at pH 7.0 for 30 minutes;
(6) Organic Solvent Tolerance: Having an enzyme activity of at least 85% in case of the treatment with ethyl acetate, butyl acetate or diisopropyl ether at pH 7.0 at 25°C for 30minutes; and
(7) Molecular Weight: Approx. 32,000 in sodium dodecylsulfate-polyacrylamide gel electrophoresis;
   further preferably to that the above wild-type carbonyl oxidoreductase mutant is the one derived from *Candida magnoliae* IFO 0705.

Furthermore, the present invention relates to an oxidoreductase mutant obtained by the above enzyme modification method, a DNA encoding said enzyme mutant, a plasmid carrying said DNA, and a transformant obtained by transformation with said plasmid.

Furthermore, the present invention relates to a process for producing of a carbonyl reductase mutant, said process comprising a step of culturing and proliferating the above transformant.

Furthermore, the present invention relates to a process for producing an optically active alcohol, said process including a step of reacting the above enzyme mutant, an enzyme having an ability of regenerating a coenzyme which said enzyme mutant depends upon and/or a mutant thereof and a carbonyl compound, and a step of harvesting the produced optically active alcohol.

### Brief Explanation of Drawings

Fig. 1 is a schematic diagram of the three dimensional structure model of a CMCRD enzyme-NADP complex, where residues at the 1 to 17 positions corresponding to the N-terminal region are excluded.
Fig. 2 shows the pH profile of CMCRD enzyme mutant S1M1.
Fig. 3 shows the thermostability of CMCRD enzyme mutant S1M1.
Fig. 4 shows the organic solvent tolerance of CMCRD enzyme mutant S1M1.
Fig. 5 shows the pH profile of CMCRD enzyme mutant S1M4.
Fig. 6 shows the thermostability of CMCRD enzyme mutant S1M4.
Fig. 7 shows the organic solvent tolerance of CMCRD enzyme mutant S1M4.

### Best Embodiment of Invention

Hereinafter, the present invention will be described in detail.

In this specification, "oxidoreductase" means an enzyme catalyzing oxidation-reduction reaction and utilizing, for example, a pyridine dinucleotide coenzyme such as NADP, NAD and the like, quinines such as ubiquinone and the like, a disulfide compound such as glutathione and the like, cytochromes, pteridine compounds, oxygen, hydrogen peroxide, etc. as electron (hydrogen) donors. In this specification, "carbonyl reductase" means an enzyme (CRD enzyme) having activity of asymmetrically reducing a carbonyl compound to produce an optically active alcohol. In this specification, amino acids, peptides and proteins are expressed using the abbreviations set forth below which are adopted by the IUPAC-IUB Committee of Biochemical Nomenclature (CBN). In addition, unless otherwise specified, amino acid sequences of a peptide and a protein will be expressed so that the N-terminal is located on the left end while C-terminal is located on the right end, and so that the first amino acid is located on the N-terminal. The linkage of amino acid residues is expressed by "-". For example, it may be expressed as Ala-Gly-Leu. In case that the same amino acid residues continues, it may be expressed, for example, as (Ala)₃, which is the same as Ala-Ala-Ala.
A=Ala=alanine, C=Cys=cysteine, D=Asp=aspartic acid, E=Glu= glutamine, F=Phe=phenylalanine, G=Gly=glycine, H=His=histidine, I=Ile=isoleucine, K=Lys=lysine, L=Leu=leucine, M=Met=methionine, N=Asn=asparagine, P=Pro=proline, Q=Gln=glutamine, R=Arg=arginine, S=Ser=serine, T=Thr=threonine, V=Val=valine, W=Trp= tryptophan, Y=Tyr=tyrosine, B=Asx=Asp or Asn, Z=Glx=Glu or Gln, and X=Xaa=arbitrary amino acid.
In order to make the reference easier in describing the produced or considered CRD enzyme mutant according to the present invention, nomenclature in the form of (original amino acid; position; and substituted amino acid) is adopted. Therefore, the substitution of aspartic acid for tyrosine at the 64-position is described as Tyr64Asp or Y64D. With respect to the description of multiple mutations, a slash sign ("/") is used for distinction. For example, S41A/Y64D represents the substitution of alanine for serine at the 41-position and that of aspartic acid for tyrosine at the 64-position.

In this specification, "a mutant" of an enzyme is a modified enzyme having an amino acid sequence obtained by substituting, inserting, deleting or modifying at least one or more amino acids of the amino acid sequence of the original enzyme and having at least some of the activities of the original enzyme.

In this specification, as examples of amino acid mutation to be utilized for the designing of a mutant, insertion, deletion or modification of an amino acid can be enumerated in addition to substitution. The substitution of an amino acid means the substitution of 1 or more, for example 1 to 20, and preferably 1 to 10, amino acids for the original peptide. The addition of an amino acid means the addition of 1 or more, for example, 1 to 20, and preferably 1 to 10, amino acids to the original peptide chain. The deletion of an amino acid means the deletion of 1 or more, for example, 1 to 20, and preferably 1 to 10, amino acids from the original peptide.

Hereinafter, the mutation of a protein amino acid for the production of a mutant will be described. Although a method for carrying out the substitution and the like of an amino acid includes chemical synthesis or a technology utilizing gene engineering in which codons of a DNA sequence encoding the amino acid are changed, the present invention is in no way restricted thereto.

In this specification, "design method" or "molecular design technique" of a mutant molecule means analyzing the amino acid sequence and the three dimensional structure of a protein or a polypeptide molecule (e.g., a natural molecule) before mutation to predict what kind of properties (e.g., catalytic activity, interaction with other molecules, etc.) individual amino acids have and to calculate an amino acid mutation suitable for producing the desired property modification (e.g., improvement of catalytic activity, improvement of protein stability, etc.). This designing method is preferably carried out using a computer. As examples of computer programs to be used for such a design method, Swiss-PDB Viewer [Swiss Institute of Bioinformatics (SIB), ExPASy Molecular Biology Server (available from http://www.expasy.ch/)] as a program for mutation introduction modeling, AMBER (D. A. Pearlman et al., AMBER 4.1, University of California, San Francisco, 1995) and PRESTO [Morikami K. et al., *Comput. Chem.*, 16, 243-248 (1992)] as programs for three dimensional structure optimization including energy minimization of a protein and computation of molecular dynamics, and Shrike (JP Patent Application No. 11-368498 (1999) : JP Patent Publication No. 2001-184381 A) as programs for computing the optimum amino acid mutation, etc. can be enumerated.

The enzyme modification method for converting the coenzyme-dependency of an oxidoreductase according to the present invention will be described below in detail, taking a CMCRD enzyme as an example. Incidentally, the present invention is in no way restricted particularly to the CMCRD enzyme to be described below, and it is obvious to a person skilled in the art that the same method can be applied to various oxidoreductases including the CRD enzyme.

In addition, the three dimensional structure of the CMCRD enzyme is unknown because experiments thereon such as structural analysis and the like, including X-ray crystal structure analysis, have not been carried out. In the present enzyme modification method, more efficient molecular design of an enzyme mutant was achieved by utilizing the three dimensional structure of an enzyme. Therefore, it is, first of all, required to obtain the three dimensional structure of a complex in which a wild-type CMCRD enzyme and a coenzyme molecule are bound, so that the prediction of the three dimensional structure of the CMCRD enzyme according to the molecular modeling and the modeling are carried out in the present invention.

The three dimensional structure of a complex with a CMCRD enzyme and a coenzyme can be constructed according to the following procedures. (Step 1) On the basis of the amino acid sequence of a CMCRD enzyme, multiple amino acid sequence alignments with enzymes which have amino acid sequence homology and whose three dimensional structures are registered in Protein Data Bank (PDB) are prepared utilizing a ClustalX program [Thompson, J. D. et al., *Nucleic Acids Res.* 22, 4673-4680 (1994)]. A protein having amino acid homology with the CMCRD enzyme to be utilized for molecular modeling can be selected by screening the amino acid sequences of the proteins registered in PDB for amino acid sequence homology by using a PASTA [Perason W. R. et al., *Genomics,* 46, 24-36 (1997)] or BLAST [Altschul, Stephen F. et al., *Nucleic Acids Res*. 25, 3389-3402 (1997)] program. A protein having a homology score (the E value on the BLAST program) of at least 1×10⁻⁵ or less, preferably of 1×10⁻¹⁰ or less with the amino acid sequence of CMCRD enzyme may be used for multiple amino acid sequence alignment as a protein having known three dimensional structure. For example, proteins having the PDB IDs 1AE1, 2AE2, 1FMC, 1CYD, 1HDC, 1YBV, 1BDB and 1EDO can be used.
(Step 2) Next, these proteins having known three dimensional structures are subjected to three-dimensional alignment by using the MAPS program [G. Lu, *J. Appl. Cryst.* (2000), 33:176-183] to modify multiple alignments previously obtained from the amino acid sequences alone on the basis of three dimensional structure analogy, whereby final multiple sequence alignments to be used for the molecular modeling can be obtained. It is desired that the modification of the sequence alignment is carried out on the basis of three dimensional structure analogy in such way that insertion or deletion does not occur in the secondary structure of α-helix, β-sheet or the like.
(Step 3) Based on the obtained sequence alignments, a protein presumed to have high three dimensional structure analogy with insertion or deletion sites as few as possible can be selected as a template protein of molecular modeling. For example, the three dimensional structures of enzymes having PDB IDs of 1AE1, 2AE2, 1FMC, 1CYD, 1HDC, 1YBV, 1BDB 1EDO and the like, and preferably those of 1YBV, 1EDO and 1FMC, can be utilized as templates. These template proteins are displayed on the three-dimensional Swiss-PDB Viewer graphics program, and, on the basis of the sequence alignment obtained in Step 2, the substitution of the amino acid residues can be carried out over the amino acid sequence of the CMCRD enzyme.
(Step 4) With respect to the insertion site and the deletion site, PDB is screened for the most suitable three dimensional structure of an analogous part and the three dimensional structure of said part is substituted, whereby a three dimensional structure model can be constructed. The molecular modeling of the insertion site or the deletion site can be carried out by screening of high resolution three dimensional structure registered in PDB, preferably protein three dimensional structure having a resolving power of 2.0Å or less for a partial three dimensional structure. This is most suitable for the partial three dimensional structure of the main chain of the template protein including the periphery of the insertion site or the deletion site. For example, a partial protein three dimensional structure having a root mean standard deviation (RMSD) of 2.0Å or less can be used when carrying out least-square superposition of the atomic coordinate of the partial three dimensional structure of the main chain of a template protein.
(Step 5) With respect to the three dimensional structure of a coenzyme NADP, which can be bound to the CMCRD enzyme, a space having a volume enabling the accommodation of NADP molecules on the three dimensional structure of the CMCRD enzyme, that is, the cleft part, is identified. Then interaction between amino acid residue group existing on the cleft part an NADP molecules is identified, whereby the atomic coordinate of the NADP can be determined.

As a method for determining the atomic coordinate of NADP, a method comprising selecting proteins binding NADP, PDB ID, 1AE1, 2AE2, 1CYD, 1YBV, and 1EDO, preferably 1YBV from the three dimensional structurally analogous proteins, and substituting the atomic coordinate of this NADP of the 1YBV can be exemplified. In addition, the atomic coordinate can be determined by firstly constructing a 3-D structural model of a CMCRD enzyme alone to which a coenzyme is not bound, then, simultaneously identifying a space having a volume enabling the accommodation of NADP molecules, that is, the cleft part with the guidance of 3-D structural energy and the interaction between amino acid residue group existing in the cleft part and the NADP molecules by using programs of molecular docking such as Autodock [(Oxford Molecular), Guex, N. and Peitsch, M. C. (1997)] after whereby the atomic coordinate of the NADP molecule can be determined. According to these methods, the three dimensional structure model of a CMCRD enzyme-NADP complex can be constructed. (Step 6) The finally constructed three dimensional structure model can be optimized in three dimensional structure by energy minimization calculation and molecular dynamics calculation. As three dimensional structure optimization programs, AMBER, PRESTO and the like can be used.

By the application of the foregoing three dimensional structure prediction and modeling techniques, a three dimensional structure model containing amino acid sequence having 30% or more homology, preferably 50% or more homology, and further preferably 70% or more homology, with that of the CMCRD enzyme which is subjected to an enzyme modification method of a CMCRD enzyme can be constructed.

Next, the identification of the active sites (catalytic site and binding site), and that of amino acid residues interacting with coenzyme molecules in the neighborhood of said active sites can be carried out according to the following method.

From the three dimensional structure model of a wild-type CMCRD enzyme-coenzyme complex and the multiple alignment of an analogous enzyme, a common amino acid sequence participating in the binding of a coenzyme (NAD and/or NADP) having high conservation in amino acid sequence, a NAD (NADP)-bound motive was found. The amino acid sequence in common between the CMCRD enzyme and the analogous CRD enzyme can be (Gly or Ala)-(Xaa)₃-(Gly, Ala or Thr)-(Ile or Leu)-(Gly, Ala or Ser)-(Xaa)₁₀-(Gly or Asn). Furthermore, by the use of three-dimensional graphics, a region neighboring the region containing this common amino acid sequence on the three dimensional structure of a complex, in other words, a region important for the binding between an enzyme and a coenzyme, can be identified. By introducing mutation into the region important for the binding between an enzyme and a coenzyme, the coenzyme-dependency can be modified. As a thus selected site, amino acid residue positions of the CMCRD enzyme at Positions 40 to 69, 87 to 92 and 225 to 228, and preferably Positions 40 to 69, can be enumerated. Further preferably, amino acid residue positions 41 to 43, 47, 63 to 66 and 69 can be enumerated as sites to be selected. In addition, using the distance as an indication, it is possible to select an amino acid residue interacting with a coenzyme molecule. For example, by making a distance within 12Å, and preferably within 8Å, from the coenzyme binding site as an indication, it is possible to select a site into which mutation is introduced.

Next, from the amino acid residues existing in the identified selection region, amino acid residues making great contribution to the binding of a NADP or NADPH coenzymes are identified by the evaluation of nonbinding interaction with coenzyme molecules by the calculation of 3-D structural energy, the calculation of electrostatic potential with attention to electrostatic interaction, and other methods. For example, the contribution of every amino acid residue in the selection region to the binding of the NADP or NADPH coenzymes can be estimated by the calculation of electrostatic potential set forth below.

First of all, a negative charge of -1 alone is placed on the phosphorus atom (where point charges of all other atoms are 0) of 2'-phosphoric acid residue of NADP (in the case of NAD, this phosphoric acid residue does not exist) in the three dimensional structure of CMCRD enzyme-NADP complex, and the electrostatic potential produced by this charge is obtained [Calculation of Electrostatic Potential, Nakamura et al., *J. Phys. Soc. Jpn.* 56, 1609-1622 (1987)]. Next, with respect to the obtained electrostatic potential, the point charge was given to atoms of all the amino acid residues, followed by calculation of the electrostatic contribution to the charge of -1 placed on phosphoric acid residues of all the residues other than 2'-phosphoric acid residue of NADP. By this calculation, the size of binding stabilization of every amino acid residue to NADP can be obtained. For example, it can be presumed that Ser 42, Tyr 64, Asn 65 and Ser 66 form positive electrostatic fields to the 2'-phosphoric acid residue of NADP, thereby contributing to the stabilization of binding to NADP.

When amino acids suitable for binding to NAD are substituted for these residues presumed to contribute to the stabilization of binding to NADP, it becomes possible to convert the coenzyme dependency of the CMCRD enzyme. Candidates for amino acid substitution can be obtained by screening the three dimensional structure models of the CMCRD enzyme-NADP complex and the CMCRD enzyme-NAD complex for the above residue sites by computer screening using, for example, a Shrike program (JP Patent Application No. 11-368498 (1999): JP Patent Application (Kokai) No. 2001-184381). In a calculative mutation experiment, the candidates for amino acid mutant can be calculated taking into account the difference between the binding energy to NADP and that to NAD and the denatured free energy as an indication of the enzyme thermostability accompanied by the amino acid substitution. Furthermore, the multiple amino acid sequence alignments at the coenzyme binding site of CRD enzyme having known three dimensional structure can be utilized as a reference for amino acid substitution.

According to the present enzyme modification method, the coenzyme dependency of an enzyme to be subjected to the regeneration of a coenzyme to be combined with the CRD enzyme also can be modified. Furthermore, it is obvious that the conversion of the substrate-specificity of the CRD enzyme becomes possible by applying the same designing technique if a molecule to be considered is changed from a coenzyme to a substrate.

In case of CMCRD enzyme, among candidates for amino acid residue substitution designed according to the present method, those for mutation at respective sites where the binding to NAD presumed to become intense are:
at the 41-position, A, G or S;
at the 42-position, A, G, S, T, R or K;
at the 43-position, A, G, S, T, Q, R or K;
at the 64-position, D;
at the 47-position, A, S, T, Y, L, Q, E, R or K;
at the 63-position, A, S, L, I, V, M, F, W, C, T, S, N or G;
at the 65-position, L, I, V, M, F, W, A, C, S or T;
at the 66-position, L, I, V, A, C, S, T, N, Q, R or K; and
at the 69-position, A, E, D or S.

Preferably, S41A, S42A, S42R, S43Q, S43G, S43R, W63I, W63L, W63V, W63F, W63M, Y64D, N65I, N65V, S66N, S66L, Y47R and A69E are enumerated as mutation candidates, and further preferably, it is possible to design mutants resulting from combinations of the above single mutations as CRD enzyme mutants.

As preferable CMCRD enzyme mutants in the present invention, the following mutants were obtained from natural CMCRD enzymes by substitution, insertion, deletion or their combination of amino acid residues:
· CMCRD enzyme mutants S41A/S42A/S43Q/W63I/Y64D/N65I/S66N;
· CMCRD enzyme mutants S41A/S42A/S43Q/W63I/Y64D/N65V/S66L;
· CMCRD enzyme mutants S41A/S42A/S43G/W63I/Y64D/N65I/S66L;
· CMCRD enzyme mutants S41A/S42A/S43R/W63I/Y64D/N65I/S66N;
· CMCRD enzyme mutants S41A/S42A/S43Q/Y47R/W63I/Y64D/N65I/S66N;
· CMCRD enzyme mutants S41A/S42A/S43R/Y47R/W63I/Y64D/N65I/S66N;
· CMCRD enzyme mutants S41A/S42R/W63I/Y64D/N65I/S66N;
· CMCRD enzyme mutants S41A/S42R/Y47R/W63I/Y64D/N65I/S66N; and
· CMCRD enzyme mutants S41A/S42A/S43Q/W63I/Y64D/N65I/S66N/A69E.

The present CRD enzyme mutants obtained according to the above methods have the physicochemical properties described in the following (1) to (3):
(1) Action:
   Acting on 4-chloroacetoacetic ethyl serving NADH as a coenzyme to produce ethyl (S)-4-chloro-3-hydroxybutyrate.
(2) Substrate Specificity:
   · Showing a strong activity to ethyl 4-chloroacetoacetate but substantially no activity to ethyl acetoacetate.
   · Showing a strong activity to 4-chloroacetoacetic ester, but having substantially no dehydrogenase activity to ethyl 4-halo-3-hydroxybutyrate.
(3) Coenzyme dependency:
   Showing a strong activity in case of serving NADH as a coenzyme, but having substantially no activity in case of serving NADPH.
   In one embodiment, the aforementioned CRD enzyme mutant further additionally has the following physicochemical properties of (4) to (7):
(4) Optimal pH: pH 4.0 - 7.0.
(5) Thermostability: Stable up to approx. 45°C when treated at pH 7.0 for 30 minutes (maintaining an enzyme activity of 75% or more when treated at 45°C for 30 minutes).
(6) Organic Solvent Tolerance: Maintaining an enzyme activity of at least 85% when treated with ethyl acetate, butyl acetate or diisopropyl ether at pH 7.0 at 25°C for 30 minutes.
(7) Molecular Weight: Approx. 32,000 by sodium dodecylsulfate-polyacrylamide gel electrophoresis.
The present CRD enzyme mutant can be produced according to a method including:
a) introducing 1 or more mutations, preferably above mutations, into a gene encoding the CRD enzyme;
b) cloning the mutation gene obtained in Step a) into a cloning vector;
c) transforming the host strain with the recombinant vector obtained in Step b);
d) culturing the host strain transformed as c) in appropriate media; and finally
e) isolating and purifying thus obtained CRD enzyme mutant.

The introduction of mutation into a gene can be carried out by utilizing one of the known *in vitro* mutation techniques, such as a site-specific mutation method or the like, although there is no restriction thereto. For example, in the case of the above CMCRD enzyme mutant, the introduction of mutation can be carried out as follows. All amino acid mutations which should be introduced exist in the approx. 170-bp restriction enzyme *Eco*O109I-*Eco*O109I site of the wild-type CMCRD enzyme gene. Therefore, the mutations can be introduced by synthesizing DNA fragments into which mutations have been introduced, corresponding to this site, followed by substituting said DNA fragments for the *Eco*O109I -*Eco*O109I site of a recombinant plasmid pNTS1 (W098/35025) containing the wild-type CMCRD enzyme gene. DNA having nucleotide sequences SEQ ID NO:1 to 9 are synthesized and subcloned in the *Pst*I site of pUC18 to produce plasmids pUCSYN181 to pUCSYN189. *Escherichia coli* JM109 or HB101 can be transformed by plasmids pUCSYN181 to pUCSYN189.. The aimed DNA fragments can be isolated by recovering plasmids from the obtained transformant, digesting the plasmids with *Eco*O109I and then subjecting the resultant to preparative polyacrylamide electrophoresis. On the other hand, after digesting the plasmid pNTS1 with *Eco*O109I, approx. 3.2-kb DNA fragments can be recovered by subjecting the digest to preparative agarose gel electrophoresis. By ligating both DNA fragments, recombinant plasmids pNTS1M1, pNTS1M2, pNTS1M3, pNTS1M4, pNTS1M5, pNTS1M6, pNTS1M7, pNTS1M8 and pNTS1M9 in which mutant genes are inserted into the *Eco*O109I - *Eco*O019I site of the pNTS1 can be constructed. The amino acid mutation to be introduced can be carried out by preparing DNA fragments containing mutation sites according to PCR and then confirming the base sequences thereof to recombinant plasmids. Since the CMCRD enzyme gene is inserted into the *Nde*I*-Eco*I site of the plasmid pNTS1, a primer carrying a base sequence upstream the *Nde*I site can be preferably used for the preparation of DNA fragments for the base sequence analysis.

The obtained recombinant plasmid carrying a CRD enzyme gene can be introduced into a particular host cell according to an ordinary method. There is no restriction to a host cell, and a microorganism, yeast, a filamentous fungus, a plant cell, an animal cell or the like may be used, with the use of *Escherichia coli* being particularly preferable. With respect to the introduction of a plasmid into a host cell, methods known to a person skilled in the art, for example, a method including a step of mixing a host cell in the competent state and a recombinant plasmid, a method including a step of introduction by conjugative transport using a helper plasmid, and others can be enumerated. For example, a transformant producing the CRD enzyme mutant can be obtained by transforming *Escherichia coli* JM109 or HB101 with the above plasmid.

In the present invention, the reduction activity to 4-chroloacetoacetic ester can be confirmed as follows.

Method for determining reduction activity to 4-chloroacetic ester: After carrying out a reaction in a reaction solution containing 0.1M potassium phosphate buffer (pH 6.5), 0.167mM NADH or NADPH, 1mM ethyl 4-chloroacetate and an enzyme at 30°C, the decrease in absorbance at 340 nm accompanied by the decrease of NADH or NADPH is determined. One U represents an amount of enzyme catalyzing a decrease of 1 µmol of NADH or NADPH for 1 minute. In case that NADH or NADPH cannot be used as a coenzyme, only a small decrease in absorbance at 340 nm is observed under this condition. In addition, the assay of a protein is carried out in accordance with the dye-binding method using a protein assay kit manufactured by Bio-Rad Laboratories, Inc.

By the application of the present enzyme modification method, it is possible to obtain a CRD enzyme mutant, which is characterized by showing a high enzyme activity when NADH is used as a coenzyme and absolutely no enzyme activity when NADPH is used. This is to say, it rigorously recognizes and utilizes NADH as a coenzyme.

The above CRD enzyme mutant shows a high reduction activity to 4-chloroacetoacetic ester but substantially no dehydrogenase activity (i.e., oxidation activity) to any optical isomers of 4-halo-3-hydroxybutyric ester. It can be said that showing substantially no dehydrogenase activity means that the CRD enzyme mutant, when it comes into contact with 4-halo-3-hydroxy-butyric ester acting as a substrate in the presence of NAD, does not show any substantial dehydrogenase activity when the rate of change per unit time in the absorbance at 340 nm accompanied by the increase or decrease of NADH is 5% or less, and preferably 1 % or less, on the relative activity base such that 100% is given to 4-chloroacetic ester.

Moreover, the above CRD enzyme mutant does not substantially show a reductase activity upon acetoacetic ester. As stated above, with regard to the index of the enzyme activity, it does not substantially show a dehydrogenase or reductase activity when the rate of change is 5%e or less, and preferably 1% or less, on the relative activity base such that 100% is given to 4-chloroacetic ester.

In the present invention, the analyses of the enzyme activity and the coenzyme-dependency of the CRD enzyme mutant can be carried out as follows, for example, using a transformant containing a DNA encoding said enzyme. In case that, as an example, the transformant is recombinant *Escherichia coli* HB101 (pNTS1M1), it can be cultured on a medium with proper composition including 50µg/ml of ampicillin, preferably on an LB medium and a 2×YT medium, and further preferably on a 2×YT medium. After harvesting the microorganism by centrifugation or the like, the harvested microorganism is suspended in a buffer with a proper pH, for example, 10 to 100mM phosphate buffer (pH 6 to 8) or 10 to 100mM Tris buffer (pH 6 to 8), and preferably 100mM phosphate buffer (pH 6.5), and then disrupted by ultrasonication, whereby a cell-free extract can be prepared. The CMCRD enzyme activity in the cell-free extract is measured according to the above assay method, and the activity of *Escherichia coli* HB101 (pNTS1) transformed with a plasmid carrying a wild-type CMCRD enzyme gene prepared according to the same method is measured. This is followed by a comparison of these activities. For example, the above CMCRD enzyme mutant reduces 4-chloroacetoacetic ester utilizing NADH as a coenzyme while the enzyme shows absolutely no reduction activity when the NADPH is used as a coenzyme. From this fact, it is confirmed that the targeted conversion (inversion) of coenzyme-dependency was succeeded.

For the extraction and the purification of an enzyme from the obtained culture, extraction and purification methods ordinarily available to a person skilled in the art can be used. For example, after centrifuging microbial cells from a culture medium, the microbial cells are suspended in an appropriate buffer and then disrupted or dissolved applying physical techniques such as use of glass beads, ultrasonication, etc., or biochemical techniques such as use of enzymes, etc. Then solid matters in said culture solution are removed by centrifugation, whereby a crude solution of the enzyme can be obtained. The above crude enzyme solution can be further purified by techniques ordinarily available to a person skilled in the art, for example, by using ammonium sulfate precipitation, dialysis, or chromatography singly or in combination. In case of chromatography, various chromatography such as hydrophobic chromatography (e.g., phenyl Sepharose), ion exchange chromatography (e.g., DEAE Sepharose), gel filtration, etc. can be used singly or in combination, whereby a CRD enzyme mutant according to the present invention can be obtained.

In the present invention, optically active alcohols can be produced from their corresponding ketone compounds by utilizing the aforementioned CRD enzyme mutant. For example, the CRD enzyme mutant according to the present invention is advantageous in industrial application because it can utilize NADH, which is cheaper and more stable than NADPH, as a coenzyme. The target enzymatic reaction can be carried out by making the enzyme molecule, a treated matter thereof, a culture containing the enzyme molecule or a transformant of a microorganism or the like producing the enzyme come into contact with a reaction solution in a viable state. Incidentally, the form of the contact between the enzyme and a reaction solution is in no way restricted to these examples. The reaction solution is such that a substrate and NADH as a coenzyme necessary for enzymatic reaction are dissolved in an appropriate solvent necessary for providing circumstances desirable for the expression of enzyme activity. As a treated matter of a microorganism containing the CRD enzyme mutant according to the present invention includes a microorganism having cell membrane of which permeability is changed by treatment with an organic solvent such as a surfactant, toluene or the like, a cell-free extract obtained by disrupting microbial bodies by glass beads, ultrasonication or enzymatic treatment, a partially purified cell-free extract, etc. are included specifically.

As a ketone to be served as a raw material in the process for producing alcohol according to the present invention, 2,3-butanedione and a 4-haloacetoacetic ester derivative, having adjacent diketones, are suitable for use. As halogens of the 4-haloacetoacetic ester derivative, bromine, chlorine and iodine are enumerated, among which chlorine is particularly suitable for use. As esters, esters of alcohol including alcohol containing straight chain, branched chain or aromatic substitution, such as methyl ester, ethyl ester, propyl ester, isopropyl ester, butyl ester, octyl ester, benzyl ester, etc. are enumerated, among which ethyl ester is the most suitable for use. As 4-haloacetoacetic acid derivatives, derivatives at the 2-position having analkyl group including a straight chain or a branched chain, or halogens such as chlorine, bromine, iodine, etc may be enumerated.

An optically active 4-halo-3-hydroxybutyric ester as a kind of optically active alcohol is obtained, for example, as follows. As a substrate, 4-haloacetoacetic ester represented by the following general formula: 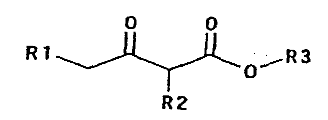 (wherein R1 is a halogen, R2 is hydrogen, and R3 is a substituted or unsubstituted alkyl group or an aryl group)
can be used. In case that R3 is an alkyl group, the R3, for example, is a methyl group, an ethyl group, a propyl group, a butyl group or an isopropyl group. In case that R3 is an aryl group, it is, for example, a phenyl group or a tolyl group. In case that the above R3 is a substituted aryl group, it is, for example, a fluorophenyl group, a chlorophenyl group or the like.

Suitably, R1 is chlorine or bromine and R3 is an alkyl group having 1 to 4 carbons. More suitably, the aforementioned substrate is methyl 4-chloroacetoacetate , ethyl 4-chloroacetoacetate, methyl 4-bromoacetoacetate or ethyl 4-bromoacetoacetate. In addition, as the aforementioned substrate, ethyl 4-iodoacetoacetate, ethyl 4-hydroxyacetoacetate, ethyl 2-chloro-3-oxobutyrate, ethyl 2-methyl-3-oxobutyrate, ethyl 4-azidoacetoacetate or the like can be used.

This 4-haloacetoacetic ester can be prepared according, for example, to the method described in JP Patent Application (Kokai) No. 61-146191 A (1986). For example, 4-haloacetoacetic ester may be prepared according to a method using diketene as a starting material, reacting a halogen thereto to prepare a 4-haloacetoacetic halide and acting an alcohol thereon, a method comprising using an acetoacetic ester as a starting material and directly halogenizing the 4-position of the acetoacetic ester, or the like.

The reaction can be carried out by adding a substrate 4-haloacetoacetic ester, a NADH coenzyme and a culture of said transformed microorganism or a treated matter thereof in water, in organic solvent having sparse solubility of water such as ethyl acetate, butyl acetate, toluene, chloroform, n-hexane or the like, or in an appropriate solvent such as a two phase system with an aqueous medium, followed by stirring under adjustment of pH. The reaction is carried out at a temperature of 10 to 70°C at a pH of 4 to 10. In addition, the concentration of a substrate to be added is 0.1 % to 90 %(w/v), which, however, can be added continuously. The reaction can be carried out by batch process or continuous process. It is also possible to carry out the reaction according to the present invention by utilizing an immobilized enzyme, a membrane reactor or the like.

Here, the treated matters of a microorganism means, for example, a crude extract, cultured microbial bodies, lyophilized microorganisms, acetone-dried microorganisms or the disrupted matters of those microbial bodies. Furthermore, they can be used in a state where an enzyme itself or a microorganism *per se* is immobilized according to a known means. Immobilization can be carried out according to methods known to a person skilled in the art (e.g., crosslinking method, physical adsorption method, inclusion method or the like).

The regeneration of NAD, which is formed from NADH accompanied by these reduction reactions, into NADH can be carried out using microorganisms having ability to reduce NAD (glycolytic pathway, C 1 compound-assimilation pathway of methylotroph, or the like). The reduction of NAD can be reinforced by adding glucose, ethanol, formic acid or the like to the reaction system. In addition, it is also possible by adding a microorganism a treated matter of said microorganism, or an enzyme having an ability to form NADH from NAD to the reaction system. For example, the regeneration of NADH can be carried out using partially or fully purified GDH, FDH, alcohol dehydrogenase, amino acid dehydrogenase, or organic acid dehydrogenase (malate dehydrogenase or the like) or the like, microorganisms containing these enzymes and treated matters thereof.

In one embodiment, as a representative NADH regeneration system for significantly decreasing the necessary amount of an expensive coenzyme, for example, a method using GDH and glucose can be enumerated. The reaction conditions, though, differ depending upon the enzyme, microorganism or treated matter thereof to be used, substrate concentration and the like, ranges a substrate concentration of approx. 0.1 % to 90 % wt%, a reaction temperature of 10 to 50°C, a pH of 4 to 8 and a reaction time of 1 to 60 hours.

When the same reaction is carried out using a culture of a transformed microorganism obtained by introducing into a single host microorganism a CRD enzyme mutant gene and a gene of an enzyme having an ability to regenerate a coenzyme upon which this enzyme depends (e.g., GDH and FDH) or the treated matter thereof, (S)-4-halo-3-hydroxybutyric ester can be produced at a lower cost because there is no necessity of separately preparing an enzyme source required for the regeneration of the coenzyme.

The 4-halo-3-hydroxybutyric ester formed by the reaction can be purified according to an ordinary method. When, for example, using a microorganism, the 4-halo-3-hydroxybu-tyric ester can be purified by removing suspensoids such as microbial bodies or the like by carrying out a treatment such as centrifugation, filtration or the like as the occasion demands, extracting the treated suspension with an organic solvent such as ethyl acetate, toluene or the like, dehydrating the extract with a dehydrating agent such as sodium sulfate or the like, removing the organic solvent under reduced pressure and then subjecting to distillation under reduced pressure, chromatography (e.g., silica gel column chromatography) or the like.

The 4-halo-3-hydroxybutyric ester can be quantified by gas chromatography. For example, the quantification of ethyl 4-chloro-3-hydroxybutyrate can be carried out by performing chromatography at 150°C using a glass column (ID 3 mm×1 m) filled with PEG-20M Chromosorb WAWDMCS (10%, 80/100 mesh; manufactured by G.L. SIENCE, Inc.) and then detecting with FID.

The determination of the optical purity of ethyl (S)-4-halo-3-hydroxybutyrate can be carried out by high performance liquid chromatography (HPLC) using a CHIRALCEL OB optical separation column (manufactured by Daicel Chemical Industries, Ltd.).

As described above, the mass production of a CMCRD enzyme mutant is possible according to the present invention. Furthermore, the utilization of this enzyme mutant provides an excellent process for producing optically active alcohols including (S)-4-halo-3-hydroxybutyric ester.

In the examples set forth below, the present invention is described in further detail. These examples are provided for the purpose of exemplifying the present invention, and the present invention is in no way restricted thereto.

### Example 1 Modeling of the three dimensional structure of CRD Enzyme originating from Candida magnoliae IFO 0705

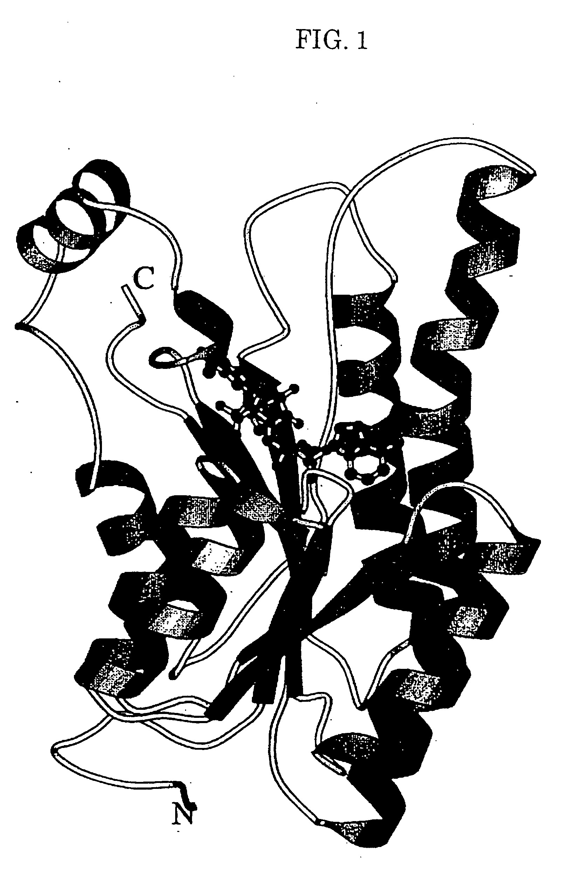

Based on the amino acid sequence (WO 98/35025) of a CRD enzyme derived from *Candida magnoliae* IFO 0705 (CMCRD enzyme), multiple amino acid sequence alignments with reduction enzymes registered in the Protein Data Bank (PDB) having known three dimensional structures (PDB ID: 1AE1, 2AE2, 1FMC, 1CYD, 1HDC, 1YBV, 1BDB) were prepared utilizing a ClustalX program [Thompson, J. D. et al., *Nucleic Acid Res.* 22, 4673-4680 (1994)]. Next, the three-dimensional alignment (conformational alignment) of these reduction enzymes having known three dimensional structures was carried out using a MAPS program [G. Lu, *J. Appl. Cryst.* (2000), 33 : 176-183]. This was followed by an examination of the correspondence to amino acid sequences of the parts having similar three dimensional structures. The above multiple alignments obtained from the amino acid sequences alone were adjusted based on this three dimensional structural information, whereby the final multiple sequence alignment to be used for modeling was obtained. The adjustment was carried out in such a manner that neither insertion nor deletion was entered in the secondary structure such as α-helix and β-sheet based on the conformational information. Based on the obtained alignment, the three dimensional structures of two enzymes 11YBV and 1FMC were selected as the basic three dimensional structures and then subjected to the substitution of amino acid residues on a Swiss-PDB Viewer three-dimensional graphic program [Swiss Institute of Bioinformatics (SIB), ExPASy Molecular Biology Server (available from http://www.expasy.ch/)]. With respect to insertion and deletion sites, the most similar partial structures were screened from PDB, and the substitution was made in said partial structure, thereby constructing an atomic coordinate. The atomic coordinate of the coenzyme NADP was constructed by using the atomic coordinate of NADP of 1YBV as a substitute. The finally constructed three dimensional structure models were optimized by calculations of energy minimization and molecular dynamics. Hereinafter, procedures for molecular modeling will be described in detail.
(1) The amino acid sequence at the 14 to 215positions of 1YBV (corresponding to the 18- to 227-position of the CMCRD enzyme) was substituted for the sequence of the CMCRD enzyme on the Swiss-PDB Viewer. With respect to the three dimensional structure of an amino acid side chain, those having no contact with peripheral residues were selected on graphics from the Rotamer Library stored in the Swiss-PDB Viewer.
(2) Using the MAPS program, the three dimensional structure of 1FMC was superposed upon that of 1YBV least square times.
(3) The amino acid sequence at the 197 to 249positions of 1FMC (corresponding to the 228- to 279-positions of the CMCRD enzyme) was substituted for the sequence of the CMCRD enzyme using the Swiss- PDB Viewer. In the amino acid substitution, the side chain three dimensional structure having the least contact energy was selected from the three dimensional structure data base of amino acid side chains (Rotamer Library) stored in the Swiss-PDB Viewer in consideration of steric hindrance with neighboring residues.
(4) The C-terminal region (the 280 to 283positions) of the CMCRD enzyme was subjected to amino acid substitution on the Swiss-PDB Viewer utilizing the main chain structure of the C-terminal region (the 242to 245positions) of 1HDC.
(5) Next, the modeling of insertion and deletion sites at the following sites was carried out. The insertion and the deletion were carried out by screening PDB for the most suitable similar partial structures and substituting the same for the partial structures, thereby constructing the atomic coordinate of the main chains.
   · Deletion of 1 residue at the 64 to 65positions of the CMCRD enzyme
   · Deletion of 2 residues at the 119 to 138positions of the CMCRD enzyme
   · insertion of 7 residues at the 154 to 171positions of the CMCRD enzyme
   · insertion of 1 residue at the 181 to 190positions of the CMCRD enzyme
   · Deletion of 1 residue at the 207 to 215positions of the CMCRD enzyme
   · Deletion of 1 residue at the 233 to 234positions of the CMCRD enzyme
(6) The former moiety of the CMCRD enzyme modeled from 1YBV after the completion of insertion and deletion, the latter moiety of the CMCRD enzyme from 1FMC and the atomic coordinate of the C-terminal region from 1HDC were merged to construct an initial three dimensional structure Model.
(7) With respect to all residues, the three dimensional structures of amino acid side chain thereof were checked again to eliminate steric hindrance and the like.
(8) Finally, using an AMBER 4.1 program (D. A. Pearlman et al., AMBER 4.1, University of California, San Francisco, 1995), the modeled three dimensional structure was optimized by calculating the energy minimization and molecular dynamics of the complex of the CMCRD enzyme (the 14- to 283-positions) and NADPH in water, which was then subjected to molecular designing. In Fig. 1, the three dimensional structure of the CMCRD enzyme-NADP complex is shown schematically.

### Example 2 Designing of CRD Enzyme Variant

From the three dimensional structure models of a wild-type CMCRD enzyme (depending upon NADPH) obtained in Example 1 and those of analogous enzymes, a NAD (NADP) binding motif (Gly-(X)₃-Gly-(Ile/Leu)-Gly-(X)₁₀-Gly) was identified in the coenzyme binding region. Furthermore, from the amino acid residues existing on this bind loop, amino acid residues greatly contributing to the NADP binding were identified by electrostatic potential calculation according to the following procedures. First, in the three dimensional structure of the CMCRD enzyme-NADP complex, a negative charge of - 1 alone (where the point charge of all the other atoms is zero) was placed on the phosphorus atom position of 2'-phosphoric acid residue of NADP, followed by finding an electrostatic potential which this charge yielded. Next, the point charge was given to all the amino acid residue atoms, followed by calculating electrostatic contribution of the found electrostatic potential to the charge of -1 placed on phosphoric acid residues of all the residues other than 2'-phosphoric acid of NADP. Amino acid residues were rearranged in the order of increasing negative contribution. The 5 top-ranked residues and 5 low-ranked residues are given in Table 1.

Ser 42, Tyr 64, Asn 65 and Ser 66, since they give a positive electric field to 2'-phosphoric acid of NADP, are presumed to contribute to the stabilization of binding to NADP. If amino acids suitable for NAD-binding are substituted for these residues, the conversion of coenzyme dependency of the CMCRD enzyme becomes possible. Candidates for amino acid substitution were selected by computer screening of the CMCRD enzyme-NADP complex three dimensional structure model with respect to the above sites using the Shrike program (JP Patent Publication (Kokai) No. 2001-184381 A). In the calculative mutation experiment, candidates for the amino acid substitution were calculated taking into account the difference in binding energy between NADP and NAD and free energy as an indication of (thermo)stability of an enzyme accompanied by the amino acid substitution. In addition, multiple amino acid sequence alignments at coenzyme-binding sites of carbonyl reductase having known three dimensional structures given in Table 2 were also utilized as a reference for amino acid substitution.

According to the present method, among the designed amino acid residue candidates fro substitution, those of high rank presumed that the binding to NAD was strengthened were S41A, S42A or S42R, S43Q, S43G or S43R, W63I, W63L, W63V, W63F or W63M, Y64D, N65I or N65V, S66N or S66L, Y47R, A69E and the like. The Cₐ carbon atom positions of these amino acid residue candidates fro substitution are at a distance of 6.9Å in the case of S41, 5.1Å in the case of S42, 6.1Å in the case of S43, 9.2Å in the case of Y47, 8.3Å in the case of W63, 5.1Å in the case of Y64, 4.5A in the case of N65, 4.3Å in the case of S66, and 8.0Å in the case of A69 from the phosphorus atom of 2'phosphoric acid residue of the coenzyme molecule NADP in the three dimensional structure of the CMCRD enzyme-NADP complex, all of which were amino acid residues within a distance of 12A from the coenzyme molecule. Finally, these mutations were combined to design the following mutants:
S41A/S42A/S43Q/W63I/Y64D/N65I/S66N mutant (S1M1),
S41A/S42A/S43Q/W63I/Y64D/N65V/S66L mutant (S1M2),
S41A/S42A/S43G/W63I/Y64D/N65I/S66L mutant (S1M3),
S41A/S42A/S43R/W63I/Y64D/N65I/S66N mutant (S1M4),
S41A/S42A/S43Q/Y47R/W63I/Y64D/N65I/S66N mutant (S1M5),
S41A/S42A/S43R/Y47R/W63I/Y64D/N65I/S66N mutant (S1M6),
S41A/S42R/W63I/Y64D/N65I/S66N mutant (S1M7),
S41A/S42R/Y47R/W63I/Y64D/N65I/S66N mutant (S1M8), and
S41A/S42A/S43Q/W63I/Y64D/N65I/S66N/A69E mutant (S1M9).

**Table 1**

| List of Amino Acid Residues Contributing NADP Binding | | | | |
|---|---|---|---|---|
| Residues Position | Residue | Contribution from Main Chain (kcal/mol) | Contribution from Side Chain (kcal/mol) | Total Contribution (kcal/mol) |
| 43 | Ser | -0.0613 | -0.2419 | -0.8582 |
| 143 | Asp | -0.0496 | -0.7200 | -0.7696 |
| 89 | Ala | -0.5817 | 0.0284 | -0.5533 |
| 63 | Trp | -0.3800 | -0.0944 | -0.4745 |
| 68 | Asp | -0.0098 | -0.4623 | -0.4721 |
| | | -Omitted- | | |
| 88 | Lys | 0.3182 | 0.5019 | 0.8201 |
| 64 | Tyr | 2.3616 | -0.0324 | 2.3292 |
| 66 | Ser | 1.5216 | 1.2305 | 2.7521 |
| 65 | Asn | 3.2160 | 1.0456 | 4.2616 |
| 42 | Ser | 1.4472 | 3.4716 | 4.9187 |

**Table 2**

| Comparison of Amino Acid Sequences of Sites Presumed to Participate in Coenzyme Binding | | | | | | | |
|---|---|---|---|---|---|---|---|
| ID | Site 1 | Site 2 | Site 3 | Site 4 | Site 5 | Site 6 | Site 7 |
| 1FMC | Ala19 | Gly20 | Ala21 | Lys25 | Asp42 | Ile43 | Asn44 |
| 1HDC | Gly14 | Ala15 | Arg16 | Ala20 | Asp37 | Val38 | Leu39 |
| 1BDB | Gly13 | Ala14 | Ser15 | Arg19 | Asp36 | Lys37 | Ser38 |
| 1DHR | Gly17 | Arg18 | Gly19 | Ser23 | Asp40 | Val41 | Val42 |
| 1YBV | Ala37 | Gly38 | Arg39 | Arg43 | Tyr60 | Asn62 | Ser63 |
| 1CYD | Ala15 | Gly16 | Lys17 | Arg21 | Thr38 | Arg39 | Thr40 |
| 2AE2 | Gly17 | Ser18 | Arg19 | Tyr23 | Thr40 | Arg41 | Asn42 |
| 1AE1 | Gly29 | Ser30 | Lys31 | Tyr35 | Ser52 | Arg53 | Asn54 |
| CMCRD | Ser41 | Ser42 | Ser43 | Tyr47 | Tyr64 | Asn65 | Ser66 |

In Table 2, "ID" means the Protein Data Bank (PDB) registration number, and the 4 kinds of carbonyl reductase given in the upper column are NAD-depending enzymes, whereas 5 kinds of carbonyl reductase, including the CMCRD enzyme, given in the lower columns are NADP-depending enzymes.

### Example 3 Making of CRD Enzyme Variant

A DNA having a nucleotide sequence of SEQ ID NO:1 was synthesized, followed by transforming *E. coli* JM109 (manufactured by Takara Shuzo Co., Ltd.) with 0.2 µg of a plasmid pUCSYN181 (manufactured by Takara Shuzo Co., Ltd.) prepared by subcloning the synthesized DNA into the *Pst*I site of pUC18. The plasmid was recovered from the obtained transformant using FlexiPrep (manufactured by Pharmacia, Inc.) and digested with *Eco*O109I, followed by subjecting the resultant to preparative polyacrylamide gel electrophoresis to isolate a 167-bp DNA fragment. On the other hand, a plasmid pNTS1 (WO098/35025) was digested with *Eco*O109I, subjected to preparative agarose gel electrophoresis to recover an approx. 3.2-kb DNA fragment, and then the DNA fragments were treated with BAP. Both DNA fragments were ligated using Takara Ligation Kit Ver. 2 (manufactured by Takara Shuzo Co., Ltd.), thereby obtaining a recombinant plasmid pNTS1M1 in which a mutant gene was inserted into the *Eco*O109I - *Eco*O109I site of the pNTS1. Using this plasmid, *E. coli* HB101 (manufactured by Takara Shuzo Co., Ltd.) was transformed, thereby obtaining *E. coli* HB101(pNTS1M1). Similarly, using synthetic DNA carrying nucleotide sequences of SEQ ID NO:2 to 9, plasmids in which mutant genes were inserted into the *Eco*O109I -*Eco*O109I site of the pNTS1 were prepared, followed by obtaining recombinant *E*. *coli* HB101 (pNTS1M2), HB101 (pNTS1M3), HB101 (pNTS1M4), HB101 (pNTS1M5), HB101 (pNTS1M6), HB101 (pNTS1M7), HB101 (pNTS1M8), and HB101 (pNTS1M9) from respective plasmids. Of the thus obtained transformants, *E. coli* HB101 (pNTS1M1), HB101 (pNTS1M2) and HB101 (pNTS1M3) were respectively deposited in International Patent Organism Depositary, National Institute of Advanced Industrial Science and Technology (1-banchi Chuo the 6^{th}, Higashi 1-chome, Tsukuba-shi, Ibaraki Prefecture, Japan) on June 22, 2001, with the accession Nos. FERM P-18388, FERM P-18389 and FERM P-18390 out of which FERM P-18388 was converted to the deposit under Budapest Treaty with the accession No. FERM BP-8059 on May 27, 2002. In addition, *E*. *coli* HB101(pNTS1M4) and HB101(pNTS1M6) were both deposited in the International Patent Organism Depositary, National Institute of Advanced Industrial Science and Technology (1-banchi Chuo the 6^{th}, Higashi 1-chome, Tsukuba-shi, Ibaraki Prefecture, Japan) on December 4, 2001, with the accession Nos. FERM P-18647 and FERM P-18648, out of which FERM P-18647 was converted to the deposit under the Budapest Treaty on May 27, 2002, with the accession No. FERM BP-8060.

The introduced amino acid mutations were confirmed by preparing DNA fragments containing mutation sites by PCR using a primer carrying a base sequence upstream of the *Nde*I site of the recombinant plasmid and comparing them with the recombinant plasmid in base sequence using an ABI373A DNA Sequencer (manufactured by Applied Biosystems).

### Example 4 Expression of CMCRD Enzyme Variant in Recombinant Escherichia coli

Recombinant *E. coli* HB101 (pNTS1M1), HB101 (pNTS1M2), HB101 (pNTS1M3), HB101 (pNTS1M4), HB101 (pNTS1M5), HB101 (pNTS1M6), HB101 (pNTS1M7), HB101 (pNTS1M8) and HB101 (pNTS1M9) obtained in Example 3 were each cultured in 2×YT media each containing 50µg/ml of ampicillin, harvested, suspended in 0.1M phosphate buffer (pH 6.5) and then ultrasonically disrupted to obtain cell-free extracts. The CMCRD enzyme activities of these cell-free extracts were determined as follows. The enzyme activities were determined by adding 1mM ethyl 4-chloroacetoacetate as a substrate, 0.167mM NADH or NADPH as a coenzyme and the enzyme to 0.1M phosphate buffer (pH 6.5), followed by determining the decrease in the absorbance at 340 nm at 30°C. The enzyme activity oxidizing 1 µmol of NADPH (NADH) to NADP⁺ (NAD⁺) for 1 minute under these reaction conditions was defined as 1 unit. The thus determined CMCRD enzyme activity in the cell-free extract was expressed as a specific activity (the enzyme activity per mg of protein contained in the extract), and the activity of *E. coli* HB101 (pNTS1) transformed with a plasmid carrying a wild-type CMCRD enzyme gene prepared in the same manner was also compared similarly. The results thereof are given in Table 3. The obtained CMCRD enzyme mutants all had such an enzyme activity such as reduced ethyl 4-chloroaceto-acetate in the presence of NADH as a coenzyme. When NADPH served as a coenzyme, those determined under the above determination conditions did not show reducing activity at all. From this fact, it can be confirmed that the conversion of coenzyme dependency of the CMCRD enzyme was attained according to the present enzyme modification method.

**Table 3**

| Relative Activities of CMCRD Enzyme Variants | | | |
|---|---|---|---|
| Enzyme | Specific Activity (U/mg) | Activity relative to NADH (%)¹⁾ | Activity relative to NADPH (%)²⁾ |
| HB101 (pNTS1) | 6.4 | 0 | 100 |
| HB101 (pNTS1M1) | 1.1 | 100 | 0 |
| HB101 (pNTS1M2) | 0.3 | 30 | 0 |
| HB101 (pNTS1M3) | 0.2 | 19 | . 0 |
| HB101 (pNTS1M4) | 3.0 | 270 | 0 |
| HB101 (pNTS1M5) | 0.4 | 33 | N.D. |
| HB101 (pNTS1M6) | 1.2 | 105 | 0 |
| HB101 (pNTS1M7) | 0.5 | 46 | N.D. |
| HB101 (pNTS1M8) | 0.1 | 7 | N.D. |
| HB101 (pNTS1M9) | 0.3 | 30 | N.D. |

| | | | |
|---|---|---|---|
| 1) Let HB101 (pNTS1M1) be 100. | | | |
| 2) Let HB101 (pNTS1) be 100. | | | |

### Example 5 Purification of CRD Enzyme Variant S1M1

Of the Escherichia coli obtained in Example 4, *E.coli* HB101 (pNTS1M1) was cultured in 2L of 2×YT medium (16 g of bacto tryptone, 10 g of bacto yeast extract, 5 g/L of salt) containing 50 µg/ml of ampicillin, followed by centrifugation to prepare microbial cells. The obtained wet microbial cells were suspended in 10mM potassium phosphate buffer (pH 6.5) containing 100µM FOIPAN (FOI, manufactured by Ono Pharmaceutical Co., Ltd.), disrupted by ultrasonication and centrifuged to remove residual microbial bodies, thereby obtaining a cell-free extract. Then, ammonium sulfate was added to this supernatant to bring it to 40% saturation and dissolved, and the formed precipitate was removed by centrifugation. Further ammonium sulfate was added to this supernatant to bring it to 70% saturation and dissolved, and the formed precipitate was collected by centrifugation. This precipitate was dissolved in 45 ml of 10mM tris-HCl buffer (pH 7.5), followed by dialyzing this against 10mM tris-HCl buffer (pH 7.5) containing 100µM FOI. The dialysate was subjected to a DEAE-Sepharose (manufactured by Pharmacia, Inc.) column (3.2×22 cm, 175 ml) previously equilibrated with the same buffer to make an enzyme mutant adsorb, and the column was washed with approx. 600 ml of the same buffer. Using the same buffer, active fractions were eluted by linear gradients of salt (0 to 0.2M). Then, 250 ml of the active fractions were collected, and ammonium sulfate was added thereto to bring it to 70% saturation and dissolved, and the formed precipitate was collected by centrifugation. This precipitate was dissolved in 30 ml of 10mM tris-HCl buffer (pH 7.5) containing 4M salt, subjected to a Phenyl-Sepharose (manufactured by Pharmacia, Inc.) column (3x20 cm, 160 ml) previously equilibrated with the same buffer to make an enzyme mutant adsorb. After washing the column with the same buffer (approx. 300 ml), active fractions were eluted by linear gradients of salt (4M to 0M) and ethylene glycol [0% to 50%(w/v)] using 10mM tris-HCl buffer (pH 7.5). Active fractions (approx. 220 ml) were collected, subjected to the exchange of buffer to 10mM tris-HCl buffer (pH 7.5) containing 0.2M salt using a ultrafiltration membrane YM-10 (manufactured by Amicon, Inc.), and concentrated to 9 ml to obtain a purified preparation of the enzyme mutant (approx. 280 mg). As a result of analyzing the purity via polyacrylamide electrophoresis (SDS-PAGE), said preparation showed a single band. Furthermore, quantification of protein was carried out according to the dye-binding method using a protein assay kit manufactured by Bio-Rad Laboratories, Inc. The specific activity of the purified enzyme S1M1 was approx. 3 U/mg.

### Example 6 Determination of Properties of CMCRD Enzyme Variant

Enzymatic properties of CMCRD enzyme mutant (S1M1) obtained in Example 5 were examined.

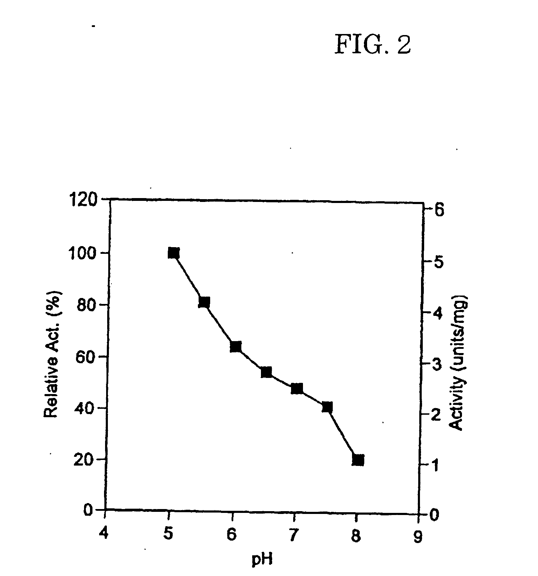

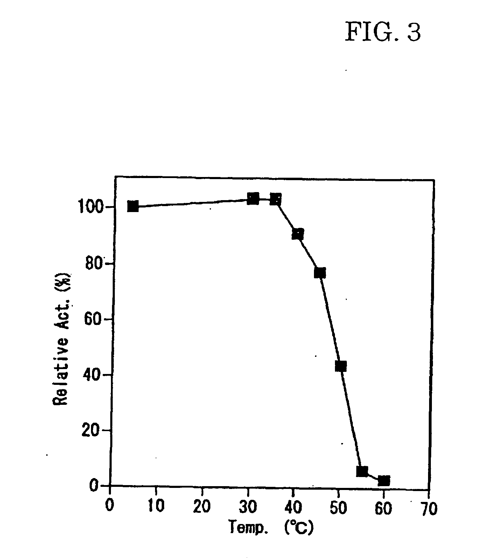

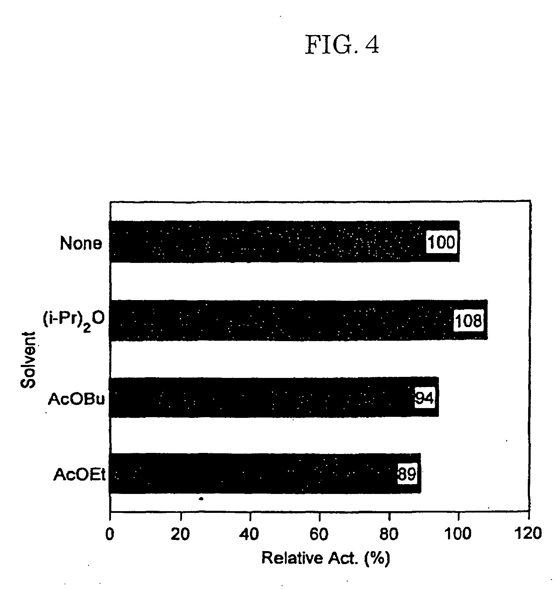

The enzyme activity measured under conditions described in Example 3.
(1) Action: The enzyme act utilizing NADH as a coenzyme on ethyl 4-chloroacetoacetate to form ethyl (S)-4-hydroxybutyrate having an optical purity of 99% e.e. or more. The ethyl (S)-4-chloro-3-hydroxybutyrate dehydrogenase activity depending upon oxidized type β-nicotinamide adenine dinucleotide (NAD⁺) was measured by monitoring the increase in absorbance at 340 nm with varying NAD⁺ from 0.33 to 3mM, ethyl (S)-4-chloro-3-hydroxybutyrate from 2 to 20mM and pH 7.0 and 8.0, but dehydrogenase activity was not observed at all.
(2) PH Profile: Using a potassium phosphate buffer as a buffer, the enzyme activity was measured within the pH range of 5.0 to 8.0 according to the above method. As a result thereof, the optimal pH acting on ethyl (S)-4-chloro-3-hydroxybutyrate was found to be in the vicinity of pH 5.0, and a higher enzyme activity was given on the acidic side (Fig. 2).
(3) Optimum Action Temperature: The activity of the present enzyme using ethyl 4-chloroacetoacetate as a substrate was determined within the temperature range of 20°C to 60°C for 1 minute to obtain an optimum temperature. As a result, the optimum temperature was found to be 40°C to 55°C.
(4) Thermostability: After treating the present enzyme at pH 7.0 for 30 minutes at temperatures of 30°C, 35°C, 40°C, 45°C, 50°C, 55°C and 60°C, the reduction activities of ethyl 4-chloroacetoacetate were determined. The results were represented by residual activities and are shown in Fig. 3, where the activity at the time of no treatment is denoted by 100. The carbonyl reductase according to the present invention gave residual activities of 85% up to 40°C and 75% or more up to 45°C.
(5) Molecular Weight: The molecular weight of the enzyme was approx. 76,000 in case of determination using a TSK-G3000SW column and, as an eluent, 0.1M potassium phosphate buffer (pH 7.0) containing 0.1M Na₂SO₄ and 0.05% NaN₃. The molecular weight of a subunit of the enzyme was determined by electrophoresis on 10% SDS-polyacrylamide gel in the presence of 2% (v/v) 2-mercaptoethanol and calculating said molecular weight from the relative mobility of the standard protein. As a result, the subunit of the present enzyme was found to have a molecular weight of approx. 32,000.
(6) Organic Solvent Tolerance: An equal quantity of ethyl acetate, butyl acetate or diisopropyl ether was added to a potassium phosphate buffer of pH 7.0 in which the present enzyme mutant was dissolved, shaken at 25°C for 30 minutes and centrifuged. The residual activity of the enzyme in the aqueous phase was determined using ethyl 4-chloroacetoacetate as a substrate. As a result, activities of 89% in the case of adding ethyl acetate, 94% in the case of adding butyl acetate and 100% in the case of diisopropyl ether were remained (Fig. 4).

### Example 7 Synthesis of (S)-4-halo-3-hydroxybutyric Acid Ester from 4-Haloacetoacetic Acid Ester by CMCRD Enzyme Variant Gene-introduced Recombinant Escherichia coli

The recombinant *E. coli* HB101 (pNTS1M1) obtained in Example 3 was inoculated in 100ml of sterilized 2×YT medium in a 500-ml Sakaguchi flask (4 flasks), cultured by shaking at 37°C for 44 hours and centrifuged to prepare microbial cells. The obtained wet microbial cells were suspended in 25 ml of 10mM phosphate buffer (pH 6.5) and then ultrasonically disrupted to obtain an enzyme solution. To 10 ml of enzyme solution, 10 ml of glucose dehyrogenase (GDH, manufactured by Amano Enzyme K.K.) solution, 7.1 g of glucose, 3.2 mg of NAD⁺, 5 g of 4-chloroacetoacetic ethyl and furthermore 5 ml of ion-exchange water and 25 ml of butyl acetate were added to prepare a solution with a gross volume of 50 ml. The solution was reacted with adjusting its pH to 6.5 using a 5M sodium hydroxide solution and with stirring at 30°C for 5.5 hours. After the completion of reaction, the reaction solution was extracted with ethyl acetate, dehydrated and then analyzed. As a result ethyl (S)-4-chloro-3-hydroxybutyrate having an optical purity of 99% e.e. was formed at a conversion ratio of 98%.

The quantification of ethyl 4-chloro-3-hydroxybutyrate was carried out by gas chromatography. Using a glass column (ID 3 mm×1 mm) filled with PEG-20M Chromosorb WAW DMCS 10% 80/100 mesh (manufactured by GL Science, Inc.), chromatography was carried out at 150°C and the detection was carried out by FID. The optical purity of ethyl (S)-4-chloro-3-hydroxybutyrate was measured by HPLC using an optical separation column CHIRALCEL OB (0.46 x 25 cm, manufactured by Daicel Chemical Industries, Ltd.). The chromatography was carried out by using a mixed solvent at a hexane : isopropanol ratio of 9:1 as the mobile phase and setting the flow rate of the mobile phase to 0.8 ml/min. The detection was carried out by monitoring the absorbance at 215 nm.

### Example 8 Purification of CRD Enzyme Variant S1M4

The *Escherichia coli* HB101 (pNTS1M4) with a high enzyme activity of Example 4 was cultured in 500 ml of 2×YT medium (16 g of bacto tryptone, 10 g of bacto yeast extract and 5 g/L of salt) containing 50 µg/ml of ampicillin and then centrifuged to prepare microbial bodies. A purified preparation of the enzyme mutant was obtained from the obtained wet microbial cells according to the same procedures as in Example 5. The purity of the purified enzyme was analyzed by polyacrylamide gel electrophoresis (SDS-PAGE)., As a result it showed a single band. Furthermore, quantitative protein analysis was carried out according to the dye-binding method using a protein assay kit manufactured by Bio-Rad Laboratories, Inc. As a result, the specific activity of the purified enzyme S1M4 was found to be approx. 10 U/mg.

### Example 9 Determination of Properties of CMCRD Enzyme Variant (2)

Enzymological properties of the CMCRD enzyme mutant (S1M4) obtained in Example 8 were examined.

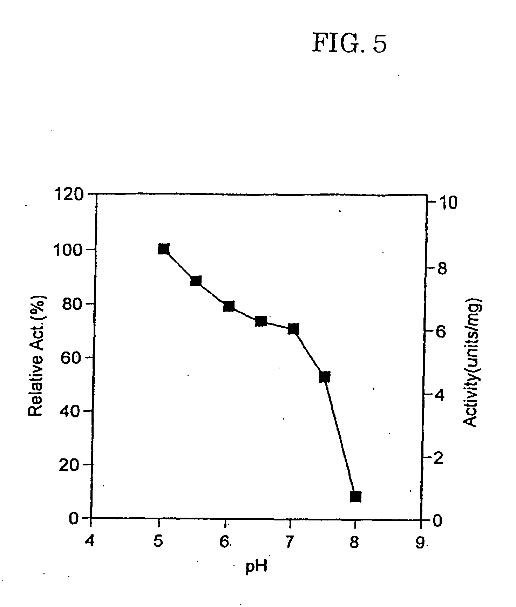

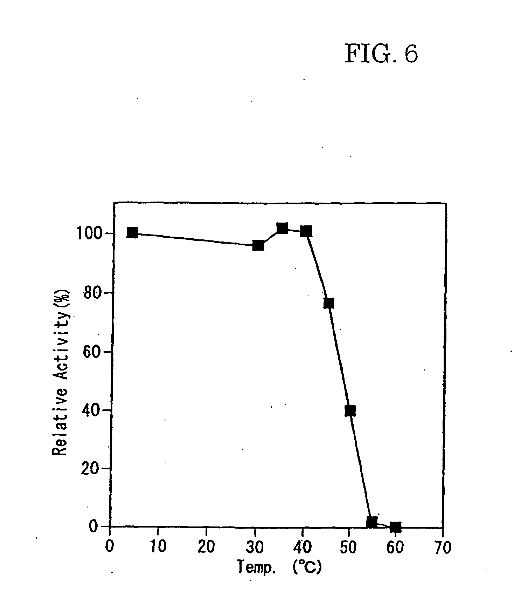

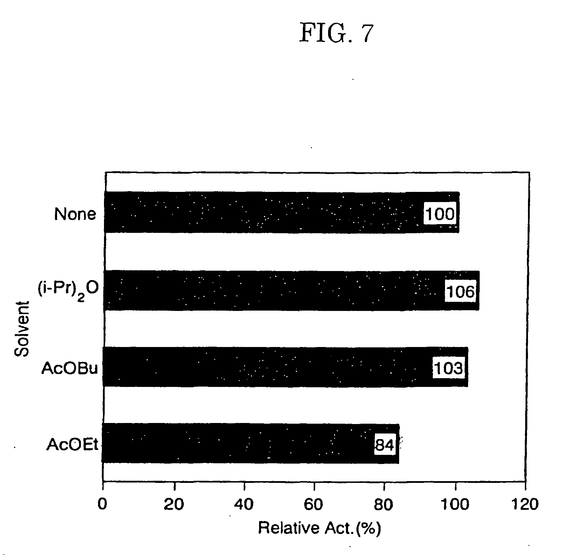

The enzyme activity was measured under the conditions described in Example 3.
(1) Action: The enzyme act utilizing NADH as a coenzyme on ethyl 4-chloroacetoacetate to form ethyl (S)-4-hydroxyburyrate having an optical purity of 99% e.e. or more. Ethyl (S)-4-chloro-3-hydroxybutyrate dehydrogenase activity depending upon oxidized type β-nicotinamide adenine dinucleotide (NAD⁺) was measured by monitoring the increase in absorbance at 340 nm with varying NAD⁺ from 0.33 to 3mM, ethyl (S)-4-chloro-3-hydroxybutyrate from 2 to 20mM and pH 7.0 and 8.0, but dehydrogenase activity was not observed at all.
(2) PH Profile: Using a potassium phosphate buffer as a buffer, the enzyme activity was measured within the pH range of 5.0 to 8.0 according to the above method. As a result thereof, the optimal pH acting on ethyl (S)-4-chloro-3-hydroxybutyrate was found to be in the vicinity of pH 5.0, and, similar to the case of the CMCRD enzyme mutant (S1M1), a higher enzyme activity was given on the acidic side (Fig. 5).
(3) Optimum Action Temperature: The activity of the present enzyme using ethyl 4-chloroacetoacetate as a substrate was determined within the temperature range of 20 to 60°C for 1 minute to obtain an optimum temperature. As a result thereof, the optimum temperature was found to be 40°C to 55°C similar to the case of the CMCRD enzyme mutant (S1M1).
(4) Thermostability: After treating the present enzyme at pH 7.0 for 30 minutes at temperatures of 30°C, 35°C, 40°C, 45°C, 50°C, 55°C and 60°C, the reduction activities of ethyl 4-chloroacetoacetate were measured. The results were represented by residual activities and shown in Fig. 6, where the activity at the time of no treatment was denoted by 100. The carbonyl reductase according to the present invention gave residual activities of 75% or more up to 45°C, similar to the case of the CMCRD enzyme mutant (S1M1).
(5) Molecular Weight: The molecular weight of the enzyme was found to be approx. 76,000 when determined using a TSK-G3000SW column and, as an eluent, 0.1M potassium phosphate buffer (pH 7.0) containing 0.1M Na₂SO₄ and 0.05% NaN₃. The molecular weight of a subunit of the enzyme was determined by electrophoresis on 10% SDS-polyacrylamide gel in the presence of 2% (v/v) 2-mercaptoethanol and calculating said molecular weight from the relative mobility of the standard protein. As a result, the subunit of the present enzyme was found to have a molecular weight of approx. 32,000.
(6) Organic Solvent Tolerance: An equal quantity of ethyl acetate, butyl acetate or diisopropyl ether was added to a potassium phosphate buffer of pH 7.0 in which the present enzyme mutant was dissolved, shaken at 25°C for 30 minutes and centrifuged. The residual activity of the enzyme in the aqueous phase was measured using ethyl 4-chloroacetoacetate. As a result, activities of 84% remained in the case of ethyl acetate was added, 103% remained in the case of butyl acetate was added and 106% remained in the case of diisopropyl ether was added. In other words, the present mutant showed organic solvent tolerance equivalent to the CMCRD enzyme mutant (S1M1) (Fig. 7).

### Industrial Applicability

According to the present invention, a method for modifying oxidoreductase characterized by controlling the binding energy of a coenzyme, an NADH-dependent carbonyl reductase mutant which is advantageous for industrial production and a DNA encoding the same, a plasmid carrying this DNA, a transformant obtained by transformation with this plasmid, and a process for producing an optically active alcohol using this enzyme mutant and/or this transformant are provided. Furthermore, it is obvious that the present enzyme modification method can be meaningfully applied to the conversion of the coenzyme dependency of an enzyme group analogous to the carbonyl reductase according to the present invention.
Incidentally, the specification of the present invention includes the specifications and the contents described in the drawings of JP patent applications Nos. 2001-200417 and 2002-006303 as priority applications of the present applications.

## Claims

1. A method for modifying an enzyme for converting the coenzyme-dependency of an oxidoreductase, **characterized by** controlling the size of the binding energy of a coenzyme molecule by substitution, insertion, deletion or the combination thereof of a single or plural arbitrary amino acid residues at the previously selected site of said oxidoreductase.

2. A method according to Claim 1, wherein the method includes a step of specifying an active site of an oxidoreductase, a step of determining an amino acid residue interacting with a coenzyme molecule in the neighborhood of said active site, and a step of carrying out mutation of said determined residue so as to control the size of the binding energy of the coenzyme molecule.

3. A method according to Claim 2, wherein said step of specifying the active site comprises predicting the three dimensional structure by the molecular modeling method, screening for the cleft part having a volume capable of accommodating the coenzyme molecule, and furthermore, comparing the amino acid sequence with analogous enzyme proteins, and extracting an amino acid residue presumed to be important for the binding of an enzyme and a coenzyme among amino acid residues constituting said cleft part.

4. A method according to Claim 2, wherein the step of determining an amino acid residue interacting with said coenzyme molecule comprises selecting amino acid residues existing within a distance of 12Å from the coenzyme molecule.

5. A method according to Claims 1 to 4, wherein said oxidoreductase utilizes a pyridine nucleotide coenzyme as a coenzyme molecule.

6. A method according to Claim 5, wherein said oxidoreductase carries (Gly or Ala)-(Xaa)₃-(Gly, Ala or Thr)-(Ile or Leu)-(Gly, Ala or Ser)-(Xaa)₁₀-(Gly or Asn) as a common amino acid sequence necessary for binding with a coenzyme molecule.

7. A method according to Claim 6, wherein the step of determining amino acid residues interacting with the coenzyme molecule further comprises selecting the amino acid residues from a region consisting of said common amino acid sequence and 15 residues each of its N-terminal and C-terminal, and preferably from a region consisting of said common amino acid sequence and 15 residues of its C-terminal.

8. An oxidoreductase mutant, which is obtained according to the method according to Claims 1 to 7.

9. A method according to Claims 1 to 7, wherein the oxidoreductase is a carbonyl reductase derived from *Candida magnoliae* IFO 0705.

10. A method according to Claim 9, **characterized by** substitution, insertion, deletion or combination thereof of amino acids in the amino acid residues of said enzyme at the 40-to 69-, 87- to 92- and 225- to 228-positions.

11. A method according to Claim 9, **characterized by** substitution, insertion, deletion or combination thereof of amino acids in the amino acid residues of said enzyme at the 41-to 43-, 47-, 63- to 66- and 69-positions.

12. A carbonyl reductase mutant, wherein the coenzyme-dependency thereof is converted by utilizing a method according to any one of Claims 9 to 11.

13. A carbonyl reductase mutant, which is obtained from a wild-type carbonyl reductase by substitution, insertion, deletion or the combination thereof of amino acid residues and has the following physicochemical properties:
(1) Action:
Acting on ethyl 4-chloroacetoacetate to produce ethyl (S)-4-chloro-3-hydoroxybutyrate by using reduced type β-nicotinamide adenine dinucleotide as a coenzyme;
(2) Substrate Specificity:
Showing a strong activity to ethyl 4-chloroacetoacetate but substantially no activity to ethyl acetoacetate, and showing a strong activity to 4-chloroacetoacetic ester but substantially no dehydrogenase activity to 4-halo-3-hydroxy-butyric ester;
(3) Coenzyme-dependency:
Showing a strong activity in case of serving reduced type β-nicotinamide adenine dinucleotide as a coenzyme but substantially no activity in case of serving reduced type β-nicotinamide adenine dinucleotide phosphate as a coenzyme.

14. A carbonyl reductase mutant according to Claim 13, which further has the following physicochemical properties of (4) to (7):
(4) Optimal pH: 4.0 to 7.0;
(5) Thermostability: Stable up to 45°C in case of the treatment at pH 7.0 for 30 minutes;
(6) Organic Solvent Resistance: Having an enzyme activity of at least 85% in case of the treatment with acetic acid ethyl, acetic acid butyl or diisopropyl ether at pH 7.0 at 25°C for 30 minutes; and
(7) Molecular Weight: Approx. 32,000 in sodium dodecylsulfate-polyacrylamide gel electrophoresis.

15. A carbonyl reductase mutant according to Claims 13 - 14, wherein said wild-type carbonyl reductase is the one derived from *Candida magnoliae* IFO 0705.

16. An enzyme mutant according to Claim 15, **characterized in that** said mutant is obtained from a wild-type carbonyl reductase by substitution, insertion, deletion or the combination thereof of amino acid residues and has
an alanine residue (A), a glycine residue (G) or a serine residue (S) at the 41-position;
an alanine residue (A) , a glycine residue (G), a serine residue (S), a threonine residue (T), an arginine residue (R) or a lysine residue (K) at the 42-position;
an alanine residue (A), a glycine residue (G), a serine residue (S), a threonine residue (T), a glutamine residue (Q), an arginine residue (R) or a lysine residue (K) at the 43-position; and an aspartic acid residue (D) at the 64-position.

17. An enzyme mutant according to Claim 16, **characterized in that** said mutant additionally has an amino acid residue selected from a group consisting of alanine (A), serine (S), threonine (T), tyrosine (Y), leucine (L), glutamine (Q), glutamic acid (E), arginine (R) and lysine (K) at the 47-position.

18. An enzyme mutant according to Claim 16 or 17, **characterized in that** said mutant additionally has an amino acid residue selected from a group consisting of alanine (A), serine (S), leucine (L), isoleucine (I), valine (V), methionine (M), phenylalanine (F), tryptophan (W), cysteine (C), threonine (T), serine (S), asparagine (N) and glycine (G) at the 63-position.

19. An enzyme mutant according to Claims 16 to 18, **characterized in that** said mutant additionally has an amino acid residue selected from a group consisting of leucine (L), isoleucine (I), valine (V), methionine (M), phenylalanine (F), tryptophan (W), alanine (A), cysteine (C), serine (S) and threonine (T) at the 65-position.

20. An enzyme mutant according to Claims 16 to 19, **characterized in that** said mutant additionally has an amino acid residue selected from a group consisting of leucine (L), isoleucine (I), valine (V), alanine (A), cysteine (C), serine (S) threonine (T), asparagine (N), glutamine (Q), arginine (R), and Lysine (K) at the 66-position.

21. An enzyme mutant according to Claims 16 to 20, **characterized in that** said mutant additionally has an amino acid residue selected from a group consisting of alanine (A), glutamic acid (E), aspartic acid (D) and serine (S) at the 69-position.

22. An enzyme mutant according to Claim 16, wherein the following mutations are introduced: S41A, S42A, S43Q, W63I, Y64D, N65I and S66N.

23. An enzyme mutant according to Claim 16, wherein the following mutations are introduced: S41A, S42A, S43Q, W63I, Y64D, N65V and S66L.

24. An enzyme mutant according to Claim 16, wherein the following mutations are introduced: S41A, S42A, S43G, W63I, Y64D, N65I and S66L.

25. An enzyme mutant according to Claim 16, wherein the following mutations are introduced: S41A, S42A, S43R, W63I, Y64D, N65I and S66N.

26. An enzyme mutant according to Claim 16, wherein the following mutations are introduced: S41A, S42A, S43Q, Y47R, W63I, Y64D, N65I and S66N.

27. An enzyme mutant according to Claim 16, wherein the following mutations are introduced: S41A, S42A, S43R, Y47R, W63I, Y64D, N65I and S66N.

28. An enzyme mutant according to Claim 16, wherein the following mutations are introduced: S41A, S42R, W63I, Y64D, N65I and S66N.

29. An enzyme mutant according to Claim 16, wherein the following mutations are introduced: S41A, S42R, Y47R, W63I, Y64D, N65I and S66N.

30. An enzyme mutant according to Claim 16, wherein the following mutations are introduced: S41A, S42A, Y43Q, W63I, Y64D, N65I, S66N and A69E.

31. A DNA encoding an enzyme mutant according to any one of Claims 22 - 30.

32. A plasmid carrying the DNA according to Claim 31.

33. A plasmid according to Claim 32, wherein said plasmid is pNTSIM1, pNTS1M2, pNTS1M3, pNTS1M4, pNTS1M5, pNTS1M6, pNTS1M7, pNTS1M8 or pNTS1M9.

34. A transformant obtained by transformation with a plasmid according to Claim 33.

35. A transformant according to Claim 34, wherein said transformant is *Escherichia coli.*

36. A transformant according to Claim 35, wherein said transformant is *E. coli* HB101 (pNTS1M1), *E. coli* HB101 (pNTS1M2), *E. coli* HB101 (pNTS1M3), *E. coli* HB101 (pNTS1M4), *E. coli* HB 101 (pNTS1M5), *E. coli* HB101 (pNTS1M6), *E. coli* HB101 (pNTS1M7), *E. coli* HB101 (pNTS1M8) or *E. coli* HB101 (pNTS1M9).

37. A process for manufacturing a carbonyl reductase mutant according to Claims 22 to 30, which comprises a step of culturing and proliferating a transformant according to Claims 34 to 36.

38. A process for manufacturing (S)-4-halo-3-hydroxybutyric ester represented by the following general formula: 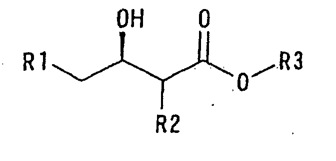 wherein R1 is a halogen atom, R2 is a hydrogen and R3 is a substituted or unsubstituted alkyl group or aryl group; and
wherein said process comprises a step of reacting 4-haloaceto-acetic ester represented by the following general formula: 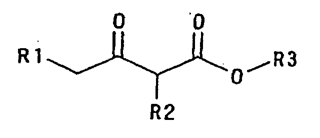 wherein R1 is a halogen atom, R2 is a hydrogen and R3 is a substituted or unsubstituted alkyl group or aryl group;
using an enzyme mutant according to any one of Claims 9 to 25, or a culture of a microorganism having an ability to produce said enzyme mutant or a treated matter of the culture.

39. A process according to Claim 39, wherein said halogen atom is chlorine or bromine and said R3 is an alkyl group having 1 to 4 carbons.

40. A process according to Claim 39, wherein said 4-haloaceto-acetic acid is methyl 4-chloroacetoacetate, ethyl 4-chloroaceto-acetate, methyl 4-bromoacetoacetate or ethyl 4-bromo-acetoacetate.

41. A process according to any one of Claims 38 to 40, wherein said microorganism is a transformant according to any one of Claims 34 to 36.

42. A process for manufacturing an optically active alcohol, which comprises a step of reacting an enzyme mutant according to any of Claims 12 - 30, an enzyme and/or its mutant having the capability of regenerating a coenzyme upon which said enzyme mutant depends and a carbonyl compound, and a step of harvesting the produced optically active alcohol.

43. A process according to Claim 42, wherein said enzyme having the capability of regenerating said coenzyme is glucose dehydrogenase and a mutant thereof.

44. A process according to Claim 42, wherein said enzyme having the capability of regenerating said coenzyme is formic dehydrogenase and a mutant thereof.

45. A process, which comprises a step of reacting a transformant obtained by transformation with a plasmid carrying a DNA encoding an enzyme mutant according to any one of Claims 12 to 30 and a DNA encoding an enzyme having the capability of regenerating a coenzyme upon which said enzyme mutant depends with a carbonyl compound and a step of harvesting the produced optically active alcohol.


SEQUENCE LISTING

<110> KANEKA CORPORATION

<120> Method for modifying enzyme and oxidoreductase variant

<130> S1 mutants

<140> ND
<141>

<150> JP 2001-200417
<151> 2001-07-02

<160> 9

<170> PatentIn Ver. 2.1

<210> 1
<211> 167
<212> DNA
<213> synthetic construct

<400> 1
ctgcaggtcc ttgacctgtt caagctgaat ggcaaggttg ctagcatcac tggcgcagca 60
cagggtattg gctacgctct ggctgaggcc ttcgcgcagg tcggcgctga cgtcgccatc 120
atcgatatca accacgacgc tactggcaag gctgaggccc tctgcag 167

<210> 2

<211> 167

<212> DNA

<213> synthetic construct

<400> 2

ctgcaggtcc ttgacctgtt caagctgaat ggcaaggttg ctagcatcac tggcgcagca 60

cagggtattg gctacgctct ggctgaggcc ttcgcgcagg tcggcgctga cgtcgccatc 120

atcgatgttc tgcacgacgc tactggcaag gctgaggccc tctgcag 167

<210> 3

<211> 167

<212> DNA

<213> synthetic construct

<400> 3

ctgcaggtcc ttgacctgtt caagctgaat ggcaaggttg ctagcatcac tggcgcagca 60

ggtggtattg gctacgctct ggctgaggcc ttcgcgcagg tcggcgctga cgtcgccatc 120

atcgatatcc tgcacgacgc tactggcaag gctgaggccc tctgcag 167

<210> 4

<211> 167

<212> DNA

<213> synthetic construct

<400> 4

ctgcaggtcc ttgacctgtt caagctgaat ggcaaggttg ctagcatcac tggcgcagca 60

cgtggtattg gctacgctct ggctgaggcc ttcgcgcagg tcggcgctga cgtcgccatc 120

atcgatatca accacgacgc tactggcaag gctgaggccc tctgcag 167

<210> 5

<211> 167

<212> DNA

<213> synthetic construct

<400> 5

ctgcaggtcc ttgacctgtt caagctgaat ggcaaggttg ctagcatcac tggcgcagca 60

cagggtattg gccgtgctct ggctgaggcc ttcgcgcagg tcggcgctga cgtcgccatc 120

atcgatatca accacgacgc tactggcaag gctgaggccc tctgcag 167

<210> 6

<211> 167

<212> DNA

<213> synthetic construct

<400> 6

ctgcaggtcc ttgacctgtt caagctgaat ggcaaggttg ctagcatcac tggcgcagca 60

cgtggtattg gccgtgctct ggctgaggcc ttcgcgcagg tcggcgctga cgtcgccatc 120

atcgatatca accacgacgc tactggcaag gctgaggccc tctgcag 167

<210> 7

<211> 167

<212> DNA

<213> synthetic construct

<400> 7

ctgcaggtcc ttgacctgtt caagctgaat ggcaaggttg ctagcatcac tggcgcacgt 60

agcggtattg gctacgctct ggctgaggcc ttcgcgcagg tcggcgctga cgtcgccatc 120

atcgatatca accacgacgc tactggcaag gctgaggccc tctgcag 167

<210> 8
<211> 167
<212> DNA
<213> synthetic construct

<400> 8
ctgcaggtcc ttgacctgtt caagctgaat ggcaaggttg ctagcatcac tggcgcacgt 60
agcggtattg gccgtgctct ggctgaggcc ttcgcgcagg tcggcgctga cgtcgccatc 120
atcgatatca accacgacgc tactggcaag gctgaggccc tctgcag 167

<210> 9
<211> 167
<212> DNA
<213> synthetic construct

<400> 9
ctgcaggtcc ttgacctgtt caagctgaat ggcaaggttg ctagcatcac tggcgcagca 60
cagggtattg gctacgctct ggctgaggcc ttcgcgcagg tcggcgctga cgtcgccatc 120
atcgatatca accacgacga aactggcaag gctgaggccc tctgcag 167